# EUROPÄISCHE PATENTSCHRIFT

(11) **EP 2 195 569 B1**
(45) Veröffentlichungstag und Bekanntmachung des Hinweises auf die Patenterteilung: **04.11.2015**
(21) Anmeldenummer: 08802726.3
(22) Anmeldetag: 30.09.2008
(51) Int. Cl.: F16M 11/04, F16M 13/02, F16M 11/18, F16M 11/20, F16G 13/16, A61B 19/00

(54) **AUFHÄNGEVORRICHTUNG**
SUSPENSION DEVICE
DISPOSITIF DE SUSPENSION

(30) Priorität: 10.10.2007 DE 102007048598
(43) Veröffentlichungstag der Anmeldung: 16.06.2010
(73) Patentinhaber: Ondal Medical Systems GmbH, 36088 Hünfeld (DE)
(72) Erfinder: STEGER, Klaus, 36132 Eiterfeld (DE)
(74) Vertreter: Graf von Stosch, Andreas
(86) Internationale Anmeldenummer: PCT/EP2008/008305
(87) Internationale Veröffentlichungsnummer: WO 2009/049769

(56) Entgegenhaltungen:
- WO-A-2006/104566
- FR-A- 1 123 037
- US-A- 898 572
- US-A- 4 568 051
- US-A1- 2003 235 320
- US-B1- 7 240 477

## Beschreibung

In medizinisch genutzten Räumen werden herkömmlicherweise Aufhängevorrichtungen eingesetzt, die es ermöglichen Geräte an den Wänden oder Decken zu befestigen. Diese Befestigungsvorrichtungen haben eine Vielzahl von Vorteilen. Zum einen ermöglichen sie eine größere Bodenfreiheit. Dies ist aus hygienetechnischen Gründen sehr vorteilhaft, da bei der Reinigung der Behandlungsräume keine Geräte verschoben werden müssen und keine Kabel und Schläuche auf dem Boden liegen. Zum anderen ermöglichen sie die freie Positionierbarkeit der Geräte innerhalb der jeweiligen Aktionsradien.

### Stand der Technik

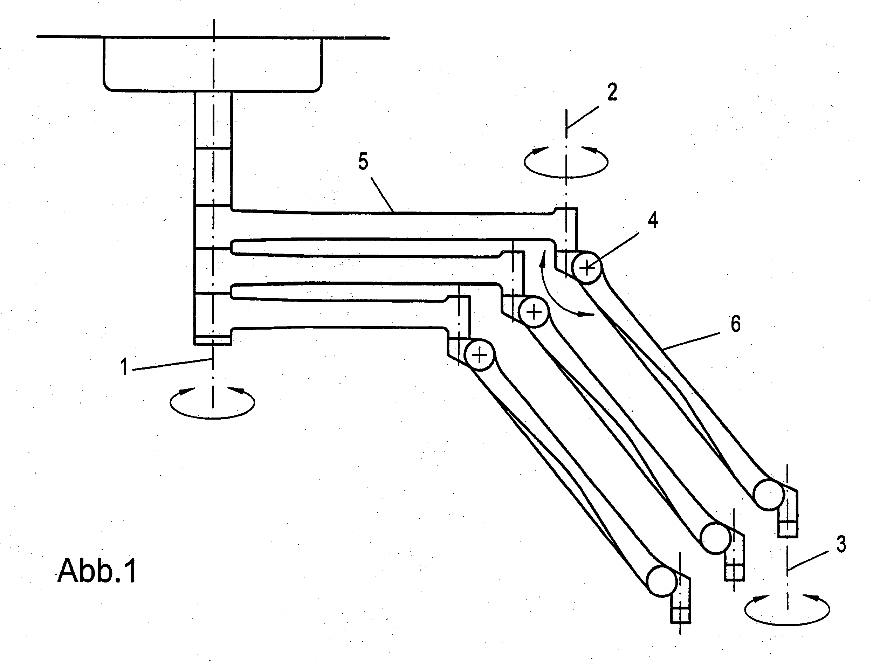

Ein Beispiel für ein bekanntes Gerät, das den oben beschrieben Zweck erfüllt, ist in Abb. 1 dargestellt. Das Gerät weist drei Achsen 1-3 für die horizontale und eine Achse 4 für die vertikale Positionierung auf. Die Vorrichtung ist durch eine vertikale Achse 1, auch Zentralachse genannt, an einer Wand oder Decke befestigt. Verbunden mit dieser Achse ist ein Ausleger 5, der horizontal beweglich ist. Je nach benötigtem Aktionsradius kann die Vorrichtung mit verschieden langen Auslegern bestückt werden. Ein Gelenk mit zwei Achsen ist am Ausleger befestigt, wobei eine Achse horizontal 4 und eine weitere Achse vertikal 2 ausgerichtet ist. Verbunden mit diesem Gelenk ist ein Federarm 6. Dieser Federarm 6 kann auf Grund der zwei Achsen 2 und 4 im anliegenden Gelenk sowohl vertikal als auch horizontal bewegt werden. Auf der dem Ausleger entgegengesetzten Seite des Federarms werden medizinische Geräte befestigt. Der Federarm hat eine weitere vertikale Achse 3. Der Federarm ist so konstruiert, dass beide Vertikalachsen 2 und 3 immer parallel zu einander bleiben.

Der Ausleger 5 hat in dieser Vorrichtung ein Hohlprofil. Die Gerätekabel sind innerhalb des Auslegers verlegt.

Die Verlegung der Kabel erfolgt üblicherweise während der Montage im Werk und ist wegen der starren und geschlossenen Vorrichtung eine schwierige Angelegenheit. Ein nachträgliches Verlegen von zusätzlichen Leitungen ist ohne eine Demontage der Vorrichtung meist nicht möglich. Als Resultat werden nachträglich installierte Leitungen oft außerhalb der Vorrichtung angebracht was. In medizinisch genutzten Räumlichkeiten ist das hygienisch bedenklich.

Des Weiteren ist zwar jede gewünschte Position innerhalb eines bestimmten Radius anfahrbar, aber der Weg zu der gewünschten Position ist nicht immer eindeutig oder intuitiv. Der Grund dafür ist das notwendige Kombinieren der Bewegungen der langen starren Ausleger mittels der vertikalen Rotationsachsen. Folglich muss der Benutzer einen Grossteil seiner Aufmerksamkeit der Bewegung des Gerätes und der einzelnen Vertikalachsen widmen

Eine Motorisierung der Bewegungen der Aufhängevorrichtung wird durch die oben beschrieben Anordnung erschwert. Dies ist auf die notwendige Automatisierung der komplizierten Bewegungsabläufe und der großen Bewegungsbereiche der einzelnen Elemente zurück zuführen. Da für jede vertikale Achse auch ein großer Rotationsradius ermöglicht werden muss, wird die Auswahl der Motoren zusätzlich erschwert.

Um den Auslegearm an die Einsatzgebiete mit ihren verschiedenen Aktionsradien anzupassen, werden hier einfach verschieden lange Auslegearme produziert. Das hat den Nachteil, dass für jede gewünschte Länge ein neues Teil produziert werden muss. Dies spiegelt sich entweder in hohe Produktionskosten und in einer geringen Auswahl von Längen wieder.

Internationale Patentanmeldung WO-2006/104566 A2 offenbart einen Ausleger für den medizinischen Bereich, welcher mehrere aneinander gereihte Glieder aufweist. Obwohl dieser Ausleger für eine interne Kabelführung ausgebildet ist, bleibt mit dieser Anordnung ein nachträgliches Verlegen der Kabel problematisch.

Es ist Aufgabe der vorliegenden Erfindung, eine verbesserte Aufhängevorrichtung bereitzustellen, die beweglicher ist, eine einfache und hygienische Kabelführung ermöglicht und preiswerter herzustellen ist.

Diese Aufgabe wird durch eine Aufhängevorrichtung gemäß Anspruch 1 gelöst. Um die oben beschriebenen Probleme zu lösen, ist der horizontale Auslegearm der vorliegenden Erfindung aus vielen aneinandergereihten Gliedern zusammengestellt. Bei den bisherigen Aufhängevorrichtungen ist der horizontale Ausleger starr und geschlossen ausgeführt. Bei der vorliegenden Erfindung wird der Ausleger durch eine Anordnung mehrer Mittelglieder, die jeweils zwei Vertikalachsen aufweisen, ersetzt. Zusätzlich zu den Mittelgliedern besitzt jede Anordnung noch ein Zentralglied zur Anbindung an einer Zentralachse sowie ein Endglied zur Befestigung der Geräte oder eines Federarmes. Alle Glieder kennzeichnen sich dadurch, dass sie ein offenes Profil, wie z.B. ein "T"- oder ein I-Profil besitzen. Die Ausbuchtungen dieses offenen Profils sind für die Aufnahme der Gerätekabel 180 geeignet und sind aus hygienetechnischen Gründen mit abnehmbaren flexiblen Abdeckungen bedeckt 190, die aus relativ starren Abdeckungsplatten 220 bestehen, die an jedem Glied mittels Stiften oder Zangen 210 befestigt sind, wobei Plattenzwischenräume 200 aus einem flexibleren Material gefertigt sind, um die Bewegungsfreiheit der einzelnen Glieder zueinander nicht zu beeinträchtigen. Die mindestens eine Ausbuchtung des jeden offenen Profils ist seitlich offen. Die Ausbuchtungen der offenprofiligen Glieder sind damit ebenfalls aneinander gereiht.

Es besteht auch die Möglichkeit, dass die Mittelglieder sowie das Endglied entsprechend ihrer Biegebelastung unterschiedlich dimensioniert sind. D.h. die Anordnung würde zum Endglied hin kleiner dimensioniert sein, da zum Endglied hin die Biegebelastung abnimmt. Hierdurch könnte der Materialeinsatz reduziert werden.

Der Vorteil bei der einfacheren und intuitiveren Bedienbarkeit entsteht durch die Anzahl der Glieder und die dadurch vermehrte Anzahl von Vertikalachsen. Auch wenn die Rotation pro Achse je nach Ausführung limitiert ist, so führt die höhere Anzahl von Achsen zu einer wesentlich einfacheren Handhabung. Der Benutzer kann nun, ohne sich auf die Aufhängevorrichtung konzentrieren zu müssen, das Gerät auf direktem Weg zu der gewünschten Position führen. Das notwendige Koordinieren der Achsen wie bei der bisherigen Aufhängevorrichtung ist nicht mehr nötig.

Im Falle einer Automatisierung wird die Auswahl der Motoren durch die kleineren Rotationsradien der einzelnen Vertikalachsen vereinfacht. Da die Anzahl der Motoren zwangsläufig steigt, kann man die gewünschte Beweglichkeit auch mit kleineren Motoren erreichen.

Die Anforderungen an bestimmte Aktionsradien kann mit dieser Erfindung durch eine unterschiedliche Anzahl von Mittelgliedern ohne großen Aufwand gewährleistet werden. Durch das zusätzliche einfügen eines Mittelgliedes, kann der Radius ohne Probleme z.B. von L1 auf L2 erweitert werden. Dies ist vor allem aus produktionstechnischen Gründen ein ausschlaggebender Vorteil, da nun die Anzahl der zu produzierenden Einzelteile minimiert wird. Die Herstellungskosten würden somit gesenkt.

Die Ausbuchtungen der offenprofiligen Glieder sind sehr gut für die Aufnahme der Gerätekabel geeignet. Die Kabel können problemlos an den Seiten der T- oder I-Profile angebracht werden. Die abnehmbaren flexiblen Abdeckungen verankern die Kabel in den Ausbuchtungen. Dadurch, dass die Abdeckungen abnehmbar sind, ist ein nachträgliches Anbringen von Kabeln ohne weiteres zu bewerkstelligen. Des Weiteren verringern die Abdeckungen die freiliegenden Stellen. Dies ist für die hohen hygienischen Anforderungen innerhalb medizinisch genutzter Räume von Vorteil ist, da sie viel einfacher zu reinigen sind und dadurch keine unhygienischen Schmutzecken entstehen.

### Kurzbeschreibung der Figuren

- Abb. 1: zeigt eine Aufhängevorrichtung, die dem Stand der Technik entspricht.
- Abb. 2: zeigt ein Mittelglied einer Aufhängevorrichtung gemäß einem bevorzugten Ausfuhrungsbeispiel der vorliegenden Erfindung.
- Abb. 3: zeigt ein Zentralglied der Aufhängevorrichtung gemäß dem bevorzugten Ausführungsbeispiel der vorliegenden Erfindung.
- Abb. 4: zeigt ein Endglied der Aufhängevorrichtung gemäß dem bevorzugten Ausführungsbeispiel der vorliegenden Erfindung.
- Abb. 5: zeigt zwei verschieden lange Gliederanordnungen der erfindungsgemäßen Aufhängevorrichtung aus der Vogelperspektive.
- Abb. 6: zeigt die perspektivische Ansicht einer Anordnung zweier verschieden langer Gliedervorrichtungen der erfindungsgemäßen Aufhängevorrichtung.
- Abb. 7: zeigt eine Gliederanordnung inklusive der Kabel und der flexiblen Abdeckungen gemäß dem bevorzugten Ausführungsbeispiel der vorliegenden Erfindung.

### Ausführliche Beschreibung des bevorzugten Ausführungsbeispiels

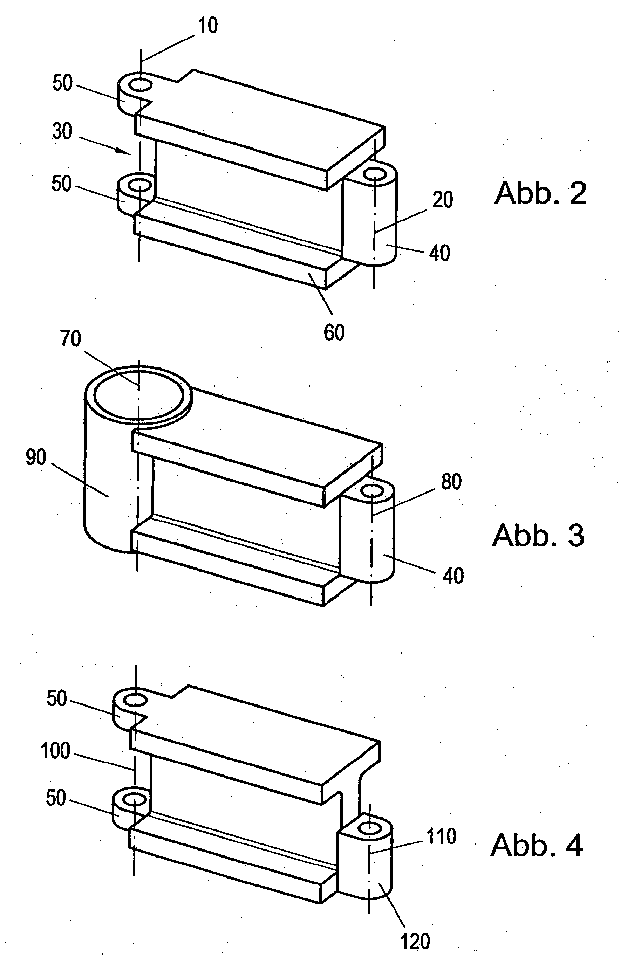

In Abb. 2 ist ein Mittelglied mit einem I-Profil gezeigt. Auf beiden Seiten des Mittelglieds befinden sich Vertikalachsen 10 und 20. Ferner weist das Mittelglied seitlich offene Ausbuchtungen 30, die sich entlang des Mittelgliedes erstrecken, zur Aufnahme einer oder mehrerer Gerätekabel. Durch Stifte, die entlang der jeweiligen Vertikalachse positioniert sind, werden die aufgereihten Mittelglieder miteinander verbunden. Angeln 40 und 50, welche die Vertikalachsen 10 und 20 teilweise umschließen, sind an den jeweiligen Enden des Mittelglieds zu finden. Die Angeln 40 und 50 sind so konstruiert, dass sie ineinander übergehen bzw. sich verzahnen, wenn zwei Glieder aneinander gereiht werden. Die beiden Angeln 50 sind schmaler konstruiert als die Angel 40. Der Grund dafür ist, dass sie sich weiter entfernt von einer horizontalen Mittelebene befinden. Die horizontale Mittelebene befindet sich zwischen der oberen und unteren Oberfläche des Mittelgliedes; sie ist parallel zu der oberen und unteren Oberfläche des Mittelgliedes angeordnet. Durch den größeren Abstand zu dieser Ebene haben die Angel 50 eine größere Hebelwirkung um den Biegekräften entgegenzuwirken. Dies hat zur Folge, dass die horizontalen Kräfte, die auf die Angeln wirken, reduziert sind. Die Angel 40 liegt direkt an der Mittelebene des Glieds. Das erhöht die horizontalen Kräfte die auf die Angel wirken. Deshalb ist die Angel 40 länger als die Angeln 50.

Dasselbe Prinzip ist auch für die Auswahl des I-Profils verantwortlich. Die Querschnittsfläche ist proportional zur Biegekraft. Der Abstand der Fläche zur Mittelebene ist ebenfalls proportional zur Biegekraft. Als Resultat, kann dieselbe Biegekraft mit einer kleineren Fläche bewerkstelligt werden, wenn die Fläche weiter von der Mittelebene entfernt ist. Das I-Profil wurde deshalb ausgewählt, weil beim Querschnitt des I-Profils ein Grossteil der Flächen den größtmöglichen Abstand zur Mittelebene hat. Das macht das I-Profil gegenüber den Biegekräften besonders resistent.

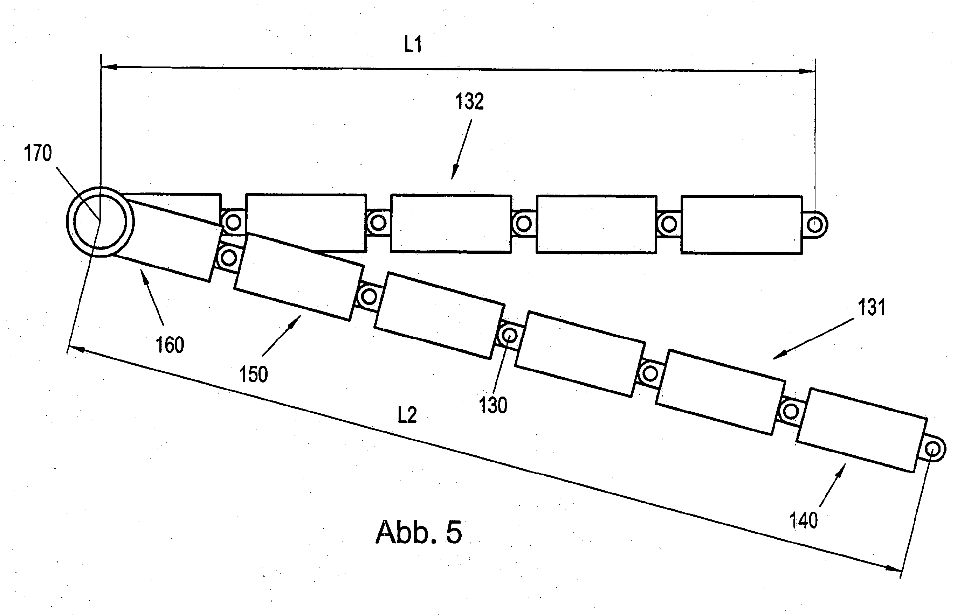

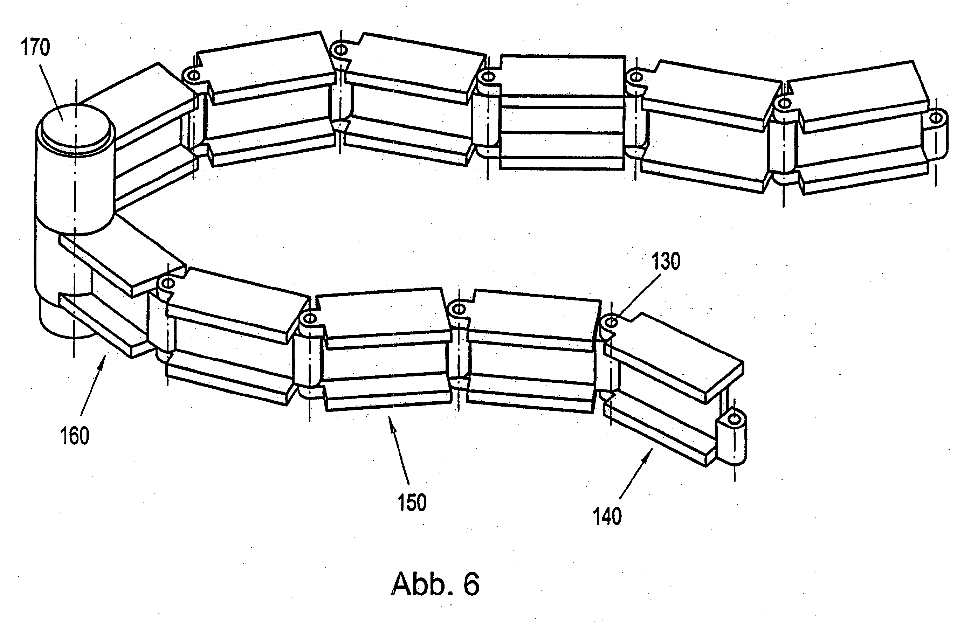

In Abb. 3 ist ein Zentralglied zu sehen. Es hat ebenfalls zwei Achsen, die Zentralachse 70 und die Vertikalachse 80. Das Ende des Zentralglieds, in dem sich die Zentralachse 70 befindet, beinhaltet Angel 90, welche in der Richtung der Zentralachse 70 ausgerichtet ist. Mit dieser Angel wird der gesamte Ausleger an einem Zapfen 170 (Abb. 6) aufgehängt. Das entgegengesetzte Ende des Zentralglieds ist genau so gestaltet wie das Ende des Mittelglieds, in dem sich die Vertikalachse 20 befindet. Es hat die gleiche Angel 40. Dieses Ende des Zentralglieds wird durch einen Stift 130 (Abb. 5) mit derjenigen Seite des Mittelglieds verbunden, die die Angel 50 (Abb. 2) beinhaltet. Der Stift bildet die Vertikalachse 80 aus. Das Mittelglied ist um die Zentralachse 80 rotierbar gelagert.

In Abb. 4 ist ein Endglied gezeigt. Es hat ebenfalls zwei Achsen, die Vertikalachse 100 und die Endachse 110. Das Ende des Endglieds, in dem sich die Vertikalachse 100 befindet, ist genauso so gestaltet, wie das Ende des Mittelglieds, in dem sich die Vertikalachse 10 befindet. Es hat die gleiche Angel 50. Dieses Ende des Endglieds wird durch einen Stift mit einem Mittelglied verbunden, und zwar mit der Seite des Mittelglieds, die die Angel 40 beinhaltet. Das andere Ende des Endglieds beinhaltet Angel 120, durch die die Endachse 110 läuft. An dieser Endachse werden mittels eines weiteren Stifts die Geräte oder der Federarm befestigt.

In Abb. 5 sind zwei, aus den verschiedenen Gliedern zusammengesetzte, Auslegearme 131 und 132 von oben gezeigt. Hier wird dargestellt, wie einfach die Länge des Auslegers, durch das Hinzufügen oder Wegnehmen eines Mittelglieds 150 verändert werden kann. An einem Ende der Abbildung ist ein Zapfen 170 dargestellt. Dieser Zapfen 170 ist normalerweise an einer Wand oder Decke befestigt. Das Zentralglied 160 ist auf der einen Seite auf den Zapfen aufgesetzt und auf der anderen Seite mit einer Reihe von Mittelgliedern 150 via Stiften 130 verbunden. Am Ende des Auslegers befindet sich das Endglied 140.

In Abb. 6 sind die gleichen Auslegearme, 131 und 132, von Abb. 5 in Perspektive zu sehen. Die Gelenke sind hier zueinander etwas verdreht um die Beweglichkeit des Auslegers zu verdeutlichen.

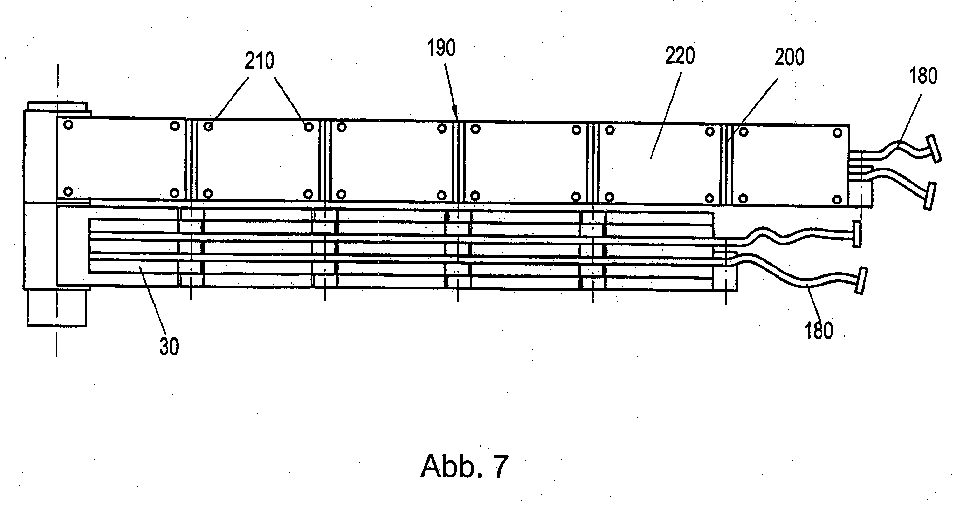

In Abb. 7 ist die Seitenansicht der Auslegearmen, 131 und 132, von Abb. 5 gezeigt. Im unteren Auslegearm ist zudem dargestellt wie die Kabel 180 innerhalb der Ausbuchtungen des I-Profils 30 verlegt sind. Die Kabel 180 können sehr leicht in die seitlich offenen Ausbuchtungen 30 der offenprofiligen Glieder gelegt werden. Im oberen Auslegearm sind auch noch die flexiblen Abdeckungen 190 zu sehen. Die Ausbuchtungen der offenprofiligen Glieder werden von den flexiblen Abdeckungen abgedeckt. Dadurch werden die darin befindlichen Kabel verankert. Die Abdeckungen bestehen aus relativ starren Abdeckungsplatten 220, die an jedem Glied mittels Stiften oder Zangen 210 befestigt sind. Die Plattenzwischenräume 200 sind aus einem flexibleren Material gefertigt, um die Bewegungsfreiheit der einzelnen Glieder zueinander nicht zu beeinträchtigen.

### Bezugszeichen

- 1.: Achse 1 (Zentralachse)
- 2.: Achse 2
- 3.: Achse 3
- 4.: Achse 4
- 5.: Ausleger
- 6.: Federarm
- 10.: Vertikalachse 10
- 20.: Vertikalachse 20
- 30.: Ausbuchtung des offenen Profils
- 40.: Angel 40
- 50.: Angel 50
- 60.: Biegekräfte tragende Fläche
- 70.: Zentralachse 70
- 80.: Vertikalachse 80
- 90.: Angel 90
- 100.: Verikalachse 100
- 110.: Endachse 110
- 120.: Angel 120
- 130.: Stift
- 131.: Ausleger 131 mit Länge L1
- 132.: Ausleger 132 mit Länge L2
- 140.: Endglied
- 150.: Mittelglied
- 160.: Zentralglied
- 170.: Zapfen
- 180.: Kabel
- 190.: flexible Abdeckung
- 200.: flexible Abdeckungszwischenräume
- 210.: Abdeckungsstifte
- 220.: Abdeckungsplatten

## Patentansprüche

1. Eine Aufhängevorrichtung zum freien Positionieren von Geräten, umfassend
mehrere offenprofilige Glieder, bestehend aus einem Zentralglied (160) zur Befestigung an einer Achse, mehreren Mittelgliedern (150) und einem Endglied (140), welche in einer horizontalen Ebene aneinander gereiht und relativ zueinander beweglich sind,
wobei jedes Profil der Glieder zumindest eine Ausbuchtung (30) aufweist und die Ausbuchtungen (30) der Profile zur Aufnahme eines Kabels geeignet sind,
wobei die Ausbuchtungen (30) der Glieder seitlich offen und derart aneinander gereiht sind, dass sie zur seitlichen Aufnahme eines Kabels entlang der Aufhängevorrichtung ausgebildet sind,
wobei die Aufhängevorrichtung ferner abnehmbare Abdeckungen (190) zum Abdecken der Ausbuchtungen (30) umfasst,
**dadurch gekennzeichnet, dass** die Abdeckungen (190) flexibel sind und aus relativ starren Abdeckungsplatten (220) bestehen, die an jedem Glied mittels Stiften oder Zangen (210) befestigt sind, wobei Abdeckungsplattenzwischenräume (200) aus einem flexibleren Material gefertigt sind, um die Bewegungsfreiheit der einzelnen Glieder zueinander nicht zu beeinträchtigen.

2. Aufhängevorrichtung nach Anspruch 1, wobei das Zentralglied (160) an einer Zentralachse (70) befestigt ist und das Zentralglied (160) um die Zentralachse rotierbar sowie entlang der Zentralachse verschiebbar ist.

3. Aufhängevorrichtung nach Anspruch 1 oder 2, wobei jedes Mittelglied (150) jeweils zwei vertikale Rotationsachsen (10, 20) sowie Angeln (40, 50), welche die Vertikalachsen (10, 20) teilweise umschließen, an seinen jeweiligen Enden aufweist, und wobei die Ausbuchtungen (30) die Kabel seitlich an den Angeln (40, 50) vorbeiführen.

4. Aufhängevorrichtung nach einem der vorhergehenden Ansprüche, wobei jedes Glied ein T-Profil, ein I-Profil oder H-Profil besitzt.

5. Aufhängevorrichtung nach einem der vorhergehenden Ansprüche, wobei das Endglied (140) den kleineren Biegebelastungen entsprechend kleiner dimensioniert ist und die Mittelglieder (150) zum Endglied hin den kleiner werdenden Biegebelastungen entsprechend kleiner dimensioniert sind, wobei die Art Dimensionierung der Widerstandsfähigkeit gegenüber den Biegbelastungen entspricht.

6. Aufhängevorrichtung nach einem der vorhergehenden Ansprüche, wobei die Länge des Gerätearmes durch das Zufügen und Wegnehmen von Mittelgliedern veränderbar ist.

## Claims

1. A suspension device for free positioning of equipment, comprising multiple openprofile elements, comprising a central element (160) for mounting on an axis, multiple intermediate elements (150), and an end element (140), which are stringed together along a horizontal plane and which are movable relative to each other, wherein each profile of the elements comprises at least one indentation (30) and the indentations (30) of the profiles are suitable for accommodating a cable, wherein the indentations (30) of the elements are laterally open and are stringed together such that they are suitable for lateral reception of a cable along the suspension device, wherein the suspension device further comprises removable coverings (190) for covering the indentations (30), **characterized in that** the coverings (190) are flexible and consisting of relatively rigid covering plates (220), which are attached to each element by means of pins or clips (210), wherein the intervals (200) between the covering plates are made of flexible material in order not to restrict the freedom of movement of the individual elements relative to one another.

2. Suspension device according to claim 1, wherein the central element (160) is mounted on a central axis (70) and the central element (160) is rotatable about the central access and also displaceable along the central axis.

3. Suspension device according to claim 1 or 2, wherein each intermediate element (150) respectively comprises to a vertical rotational access (10, 20) as well as hinges (40, 50) at its respective ends, which at least partially surround the vertical axis (10, 20), and wherein the indentations (30) bypass the cables laterally at the hinges (40, 50).

4. Suspension device according to any one of the preceding claims, wherein each element can have a T-profile, an I-profile or an H-profile.

5. Suspension device according to any one of the preceding claims, wherein the end element (140) is dimensioned smaller corresponding to the lower bending loads and the intermediate elements (150) are dimensioned smaller towards the end elements corresponding to reducing bending loads, wherein the type of dimensioning corresponds to the resistance with respect to the bending loads.

6. Suspension device according to any one of the preceding claims, wherein the length of the apparatus arm is variable by the insertion and removal of intermediate elements.

## Revendications

1. Dispositif de suspension pour le libre positionnement d'appareils, comprenant plusieurs organes à profil ouvert se composant d'un organe central (160) pour la fixation à un axe, de plusieurs organes médians (150) et d'un organe d'extrémité (140), qui sont à la suite les uns des autres dans un plan horizontal et mobiles les uns par rapport aux autres,
chaque profil des organes présentant au moins un renflement (30) et les renflements (30) des profils étant appropriés pour la réception d'un câble,
les renflements (30) des organes étant latéralement ouverts et à la suite les uns des autres de telle manière qu'ils sont réalisés pour la réception latérale d'un câble le long du dispositif de suspension,
le dispositif de suspension comportant de plus des recouvrements amovibles (190) pour le recouvrement des renflements (30),
**caractérisé en ce que** les recouvrements (190) sont flexibles et sont constitués de plaques de recouvrement (220) relativement rigides qui sont fixées sur chaque organe à l'aide de goupilles ou pinces (210), des espaces intermédiaires de plaque de recouvrement (200) étant fabriqués en un matériau plus flexible afin de ne pas entraver la liberté de mouvement des organes individuels les uns par rapport aux autres.

2. Dispositif de suspension selon la revendication 1, l'organe central (160) étant fixé sur un axe central (70) et l'organe central (160) étant rotatif autour de l'axe central ainsi que déplaçable le long de l'axe central.

3. Dispositif de suspension selon la revendication 1 ou 2, chaque organe médian (150) présentant respectivement deux axes de rotation verticaux (10, 20) ainsi que des gonds (40, 50), qui entourent en partie les axes verticaux (10, 20), sur ses extrémités respectives, et les renflements (30) faisant passer les câbles latéralement devant les gonds (40, 50).

4. Dispositif de suspension selon l'une quelconque des revendications précédentes, chaque organe possédant un profil en T, un profil en 1 ou un profil en H.

5. Dispositif de suspension selon l'une quelconque des revendications précédentes, l'organe d'extrémité (140) étant dimensionné plus petit de manière à correspondre aux charges de flexion plus petites et les organes médians (150) étant dimensionnés plus petits vers l'organe d'extrémité de manière à correspondre aux charges de flexion qui diminuent, le type de dimensionnement correspondant à la capacité de résistance par rapport aux charges de flexion.

6. Dispositif de suspension selon l'une quelconque des revendications précédentes, la longueur du bras d'appareil étant modifiable par l'ajout et le retrait d'organes médians.
